# EUROPEAN PATENT APPLICATION

(11) **EP 2 985 280 A1**
(43) Date of publication of application: **17.02.2016**
(21) Application number: 15180463.0
(22) Date of filing: 11.08.2015
(51) Int. Cl.: C07D 307/50

(54) **A PROCESS FOR SYNTHESIS OF FURFURAL OR DERIVATIVES THEREOF**

(30) Priority: 11.08.2014 IN MU25922014
(71) Applicant: Reliance Industries Limited, Mumbai 400 021 Maharashtra (IN)
(72) Inventor: VISHNUDEV, SHARMA RAJESH, 400101 Mumbai (IN); ONKAR, KATOLE SURAJ, 400708 Mumbai (IN); BALKISAN, DHOOT SHRIKANT, 400706 Mumbai (IN); UPPARA, PARASU VEERA, 400614 Mumbai (IN)
(74) Representative: Heinze, Ekkehard

(57) **Abstract**

The present disclosure relates to a process for preparing furfural or its derivatives including but not limited to 5-hydroxymethyl furfural (HMF) by dehydrating sugars in presence of organic catalyst and promoter. More particularly, the present disclosure provides a process for dehydration of monosaccharide using a homogeneous catalyst and promoter in presence of homogeneous mixture of solvent system to yield furfural derivative including but not limited to 5-hydroxymethyl furfural (HMF). The said process is simple, fast, economical and offer several advantages including but not limited to high sugar conversion and significantly low by-product formation, mild reaction/operating conditions, simple product isolation procedure and high product yield.

## Description

### TECHNICAL FIELD

The present disclosure relates to the field of chemical sciences. The present disclosure generally relates to a simple, economic and effective process for the preparation of heterocyclic organic compounds in general, and synthesis of heterocyclic aldehyde derivatives in particular. The present disclosure provides a process for synthesizing heterocyclic aldehyde derivatives, preferably furfural or derivatives thereof including but not limited to 5-hydroxymethyl furfural (HMF). More particularly, the present disclosure relates to a process which involves selective dehydration of monosaccharide to yield furfural derivatives including but not limited to 5-hydroxymethyl furfural (HMF).

### BACKGROUND

Nature produces about 170 billion metric tons of biomass per year by photosynthesis wherein biomass carbohydrates are the most abundantly available biomass. They are over functionalized with hydroxyl groups and thus need to be defunctionalized by efficient technologies to produce alcohols, aldehydes, ketones, carboxylic acids and esters by either chemical or biochemical route. Most of the research activities in this regard concentrate on identifying attractive chemical transformation routes to convert biomass into organic bulk chemicals, and to develop economically feasible processes on commercial scale.

Furfural derivatives, such as 5-hydroxymethyl furfural (HMF) obtained from renewable resources acts as a key compound that bridges the gap between biomass and bio-refinery. HMF has been of great interest since the last decade of the 19th century as wide range of chemical intermediates and end products can be produced from HMF that could be used in polymer, fuel and pharmaceutical industries. HMF possesses a high potential industrial demand, and hence it is also called as 'sleeping giant chemical' obtained from renewable resources. Regarding some of the potential applications, HMF can serve as precursor for production of FDCA (furan dicarboxylic acid) which can be used in the synthesis of PEF (polyethylene furanoate), liquid alkanes (C7-C15) that serve as diesel fuel components and pharmaceutical intermediates.

The complete conversion of sugars, and an efficient extraction of HMF is required for developing commercially viable technology. Despite the large amount of research inputs, there has not been much economically successful HMF technology. A few reasons for the same are low selectivity to HMF, requirement of strong acids which need neutralization, need for expensive heterogeneous catalyst and high boiling polar solvent which makes separation process more expensive. Further, the currently known processes of HMF synthesis through sugar route are carried out using mineral acids (such as HCl, H₃PO₄, H₂SO₄), organic acids (such as oxalic acid, levulinic acid, maleic acid, p-toluenesulfonic acid), ion exchange resins, zeolites, transition metal ions, solid metal phosphates and so on which are associated with numerous drawbacks. For instance, mineral acid catalysts give high fructose conversion (70-90%) but are associated with low selectivity to HMF yield (40-50%). Another major disadvantage of mineral acid catalysts is corrosion of reactor, separation of the product and large amount of acid waste. Similarly, solid acid catalysts such as, H-form zeolite and metal phosphates (vanadyl phosphate) although result in suitable HMF yield, they give low fructose conversion (30- 50%). Further, use of heterogeneous solid acid catalysts demonstrate good results over homogenous acids in terms of recycle and material corrosion, however solid acid catalysts result in low conversions (30-60%) and require long reaction times, for instance, greater than 2 hours. There are several patents available such as, US 2750394, US 2917520, US 2929823, US 3118912, US 4339387, US 4740605A which mention the synthesis and application of HMF. However, the methods disclosed in these patents suffer from various drawbacks such as, low conversion, low yield and problem associated with HMF isolation.

Thus, it can be observed that the existing methods of synthesizing furfural or its derivatives (such as HMF) suffer from various drawbacks and hence, there is an immense need for improved, faster, economical and high yielding synthetic process for obtaining said compounds.

### SUMMARY OF THE DISCLOSURE

The present disclosure relates to a process for preparing furfural or derivatives thereof, said process comprising act of dehydrating sugar in presence of organic acid catalyst. More particularly, the disclosure relates to a process for preparing furfural derivatives which comprises act of dehydrating sugar in presence of organic acid catalyst.

In non-limiting embodiments, the furfural derivative includes but is not limited to 5-hydroxymethylfurfural (HMF).

In non-limiting embodiments, the present disclosure provides a method for synthesizing 5-hydroxymethylfurfural (HMF) by dehydration of sugar including but not limited to monosaccharide. In another non-limiting embodiment, the monosaccharide includes but is not limited to fructose. In a preferred embodiment, the monosaccharide is fructose. In other embodiments, the HMF synthesis involves selective dehydration of monosaccharide in a homogeneous solvent system using homogeneous catalyst and promoter. In a non-limiting embodiment, the homogeneous catalyst includes but is not limited to organosulfur compound. In still another non-limiting embodiment, the promoter includes but is not limited to ionic compound/salt. In yet another non-limiting embodiment, the homogeneous solvent system includes but is not limited to a homogeneous mixture of polar solvent. In another non-limiting embodiment, the solvent system includes but is not limited to a homogeneous mixture of acetone: water.

More particularly, the present disclosure provides a method for synthesizing 5-hydroxymethylfurfural (HMF) by selective dehydration of monosaccharide in presence of organosulfur compound and ionic compound/salt in a homogenous solvent system. In a preferred embodiment, the method comprises dehydrating fructose in presence of alkyl sulfonic acid and ionic compound/salt in homogenous solvent system. In an exemplary embodiment, the alkyl sulfonic acid includes is methane sulfonic acid. In another preferred embodiment, the ionic compound/salt is sodium chloride, potassium chloride, or a combination thereof. In yet another preferred embodiment, the homogeneous solvent system is acetone: water.

The present disclosure also relates to the use of homogeneous solvent system, organosulfur compounds and ionic compounds/salts for synthesis of furfural derivatives including but not limited to 5-hydroxymethyl furfural (HMF).. In a non-limiting embodiment, the organosulfur compound includes but is not limited to alkyl sulfonic acid. In another non-limiting embodiment, the ionic compound includes but is not limited to sodium salt. In yet another non-limiting embodiment, the solvent system includes but is not limited to homogeneous mixture of polar solvent. In another non-limiting embodiment, the solvent system includes but is not limited to homogeneous mixture of acetone:water.

### DETAILED DESCRIPTION

The present disclosure relates to a process for preparing furfural or derivatives thereof, said process comprising act of dehydrating sugars in presence of organic acid catalyst.

In a non-limiting embodiment, the furfural or its derivative includes but is not limited to 5-hydroxymethylfurfural (HMF).

In a non-limiting embodiment, the present disclosure provides the synthesis of furfural derivative including but not limited to HMF comprising step of selective dehydration of monosaccharide in presence of homogeneous catalyst and promoter.

In a non-limiting embodiment of the present disclosure, the monosaccharide employed in the process includes but is not limited to fructose. In a preferred embodiment of the present disclosure, the monosaccharide employed in the process is fructose.

In another non-limiting embodiment of the present process, the homogeneous catalyst includes but is not limited to organosulfur compound.

In yet another non-limiting embodiment, the promoter employed in the present process includes but is not limited to ionic compound/salt, wherein said ionic compound is selected from a group comprising sodium chloride and potassium chloride, or a combination thereof.

In an exemplary embodiment of the present disclosure, the process of preparing furfural derivative including but not limited to HMF comprises act of selective dehydration of fructose using a homogeneous catalyst and a promoter. In a non-limiting embodiment, the homogeneous catalyst includes but is not limited to organosulfur compound. In a preferred embodiment, the organosulfur compound includes but is not limited to alkyl sulfonic acid. In a more preferred embodiment, the alkyl sulfonic acid includes but is not limited to methane sulfonic acid, ethane sulfonic acid and propane sulfonic acid, or any combination thereof. In a most preferred embodiment, the organosulfur compound is methane sulfonic acid.

In another non-limiting embodiment of the above mentioned process, the promoter includes but is not limited to ionic compound/salt.

In a preferred embodiment of the above mentioned process, the ionic compound is metal halide, preferably alkali metal halide of formula I

M⁺X⁻ Formula I

wherein,
'M' is an alkali metal selected from a group comprising lithium, sodium, potassium, rubidium, and caesium; and
'X' is a halide selected from a group comprising fluorine, chlorine, bromine and iodine.

In another preferred embodiment, the ionic compound/salt is sodium salt. In a more preferred embodiment, the sodium salt includes but is not limited to sodium chloride and potassium chloride or any combination thereof. In an exemplary embodiment, the ionic compound/salt is sodium chloride.

In a non-limiting embodiment, the monosaccharide is a concentration ranging from about 1 to 30 wt% , preferably about 10 to 15 wt%.

In a non-limiting embodiment, the homogeneous catalyst employed in the process of preparing furfural derivative including but not limiting to HMF is present at a concentration ranging from about 0.1 to 1.5 wt% with respect to total reaction volume. In a preferred embodiment, the concentration of homogeneous catalyst ranges from about 0.4 to 0.8 wt% with respect to total reaction volume.

In a non-limiting embodiment, the promoter employed in the process of preparing furfural derivative including but not limited to HMF is present at a concentration ranging from about 0.1 to 5.0 wt % with respect to total reaction volume. In a preferred embodiment, the concentration of promoter ranges from about 1.25 to 2.5 wt% with respect to total reaction volume.

In a non-limiting embodiment of the present disclosure, the process of preparing furfural derivative including but not limited to HMF is carried out in a solvent system. In a preferred embodiment, the solvent system includes but is not limited to homogeneous solvent system.

In another non-limiting embodiment, the solvent includes but is not limited to a homogeneous mixture of polar solvent. In another non-limiting embodiment, the solvent includes but is not limited to a homogeneous mixture of acetone: water, methanol: water, ethanol: water and isopropyl alcohol: water, or any combination thereof. In an exemplary embodiment, the solvent system is acetone: water.

In a non-limiting embodiment of the present disclosure, the aforesaid homogeneous solvent mixture employed in the process of preparing furfural derivative including but not limited to HMF is present in a ratio ranging from about 9:1 to 1:9. In a preferred embodiment, the said homogeneous solvent mixture is present in a ratio ranging from about 7:3 to 3:7. In a most preferred embodiment, the homogeneous solvent mixture is acetone and water present in a ratio of about 7:3.

In a non-limiting embodiment, the process of preparing furfural derivative including but not limited to HMF is carried out at a temperature ranging from about 115 °C to 130 °C. In a preferred embodiment, the process of preparing furfural derivative including but not limited to HMF is carried out at a temperature raging from about 120 °C to 130°C. In a most preferred embodiment, the process of preparing furfural derivative including but not limited to HMF is carried out at a temperature of about 125 °C.

In a non-limiting embodiment, the synthesis of furfural derivative including but not limited to HMF is completed within a time-period ranging from about 15 minutes to about 30 minutes. In a preferred embodiment, the said process of synthesis of furfural derivative including but not limited to HMF is completed within a time-period ranging from about 15 minutes to 20 minutes.

In another non-limiting embodiment of the present disclosure, the process of preparing furfural derivative including but not limited to HMF comprises act of dehydrating monosaccharide in a homogeneous solvent mixture in presence of alkyl sulfonic acid and ionic compound/salt. In a non-limiting embodiment, the monosaccharide includes but is not limited to fructose. In a preferred embodiment, the monosaccharide is fructose. In another non-limiting embodiment, the alkyl sulfonic acid includes but is not limited to methane sulfonic acid, ethane sulfonic acid and propane sulfonic acid, or any combination thereof. In a preferred embodiment, the alkyl sulfonic acid is methane sulfonic acid. In yet another non-limiting embodiment, the ionic compound/salt includes but is not limited to sodium chloride and potassium chloride, or a combination thereof. In a preferred embodiment, the ionic compound/salt is sodium chloride. In still another non-limiting embodiment, the homogeneous solvent mixture includes but is not limited to a mixture of acetone and water.

In an exemplary embodiment of the present disclosure, the process of preparing HMF comprises act of selective dehydration of fructose in a homogeneous solvent mixture using alkyl sulfonic acid (catalyst) and sodium salt (promoter). In a non-limiting embodiment the alkyl sulfonic acid includes but is not limited to methane sulfonic acid, ethane sulfonic acid and propane sulfonic acid, or any combination thereof, preferably methane sulfonic acid. In another non-limiting embodiment, the sodium salt includes but is not limited to sodium chloride and potassium chloride, or a combination thereof, preferably sodium chloride. In yet another non-limiting embodiment, the preferred homogeneous solvent mixture includes but is not limited to a combination of acetone and water.

In another exemplary embodiment of the present disclosure, the process of preparing HMF comprises act of selective dehydration of fructose in acetone: water solvent system using methane sulfonic acid and sodium chloride.

In a non-limiting embodiment, the conversion of monosaccharide in the present process ranges from about 70 % to 100 %. In a preferred embodiment, the conversion of monosaccharide ranges from about 80 % to 98 %. In a non-limiting embodiment, the monosaccharide includes but is not limited to fructose.

In another non-limiting embodiment of the present process, the yield of furfural derivative including but not limited to HMF ranges from about 65 % to 88 %. In another non-limiting embodiment, the yield of furfural derivative, more particularly HMF, ranges from about 65 % to 86 %. In an exemplary embodiment, the yield of furfural derivative, more particularly HMF is about 86±2%.

In a non-limiting embodiment of the present process, the synthesized furfural derivative including but not limited to HMF is isolated and optionally purified using methods selected from a group comprising addition of base, quenching, solvent extraction, filtration, evaporation and crystallization, or any combination of acts thereof. In a preferred embodiment, the furfural derivative including but not limited to HMF is isolated by simple solvent extraction method. In an exemplary embodiment, the furfural derivative, particularly HMF is isolated by adding a known quantity of sodium carbonate or sodium bicarbonate to the reaction mass to neutralize the catalyst. Thereafter, the furfural derivative is extracted from reaction mass by using ethyl acetate. Then ethyl acetate is evaporated under reduced pressure in rota vac to obtain isolated furfural derivative.

The present process of synthesizing furfural derivative including but not limited to HMF is simple, economical and offer several advantages including but not limited to selective dehydration of monosaccharide to furfural derivative, high monosaccharide conversion, simple & easy procedure of extraction/purification of product, significantly low by-product formation, mild reaction/operating conditions and devoid of high boiling solvent(s), reduced process time, and high product yield.

Additional embodiments and features of the present disclosure will be apparent to one of ordinary skill in art based upon description provided herein. The embodiments herein provide various features and advantageous details thereof in the description. Descriptions of well-known/conventional methods and techniques are omitted so as to not unnecessarily obscure the embodiments herein. The examples provided herein are intended merely to facilitate an understanding of ways in which the embodiments herein may be practiced and to further enable those of skill in the art to practice the embodiments herein. Accordingly, the following examples should not be construed as limiting the scope of the embodiments herein.

### EXAMPLES

### Experimental setup/Materials employed for arriving at the examples of the present disclosure

The experiments are carried out in 100 ml Parr autoclave reactor equipped with pitch blade turbine impeller for agitation. The reagents/chemicals employed in experiments are fructose, methane sulfonic acid (MSA), sodium chloride, potassium chloride, and acetone-distilled water mixture.

In order to study the dehydration of fructose to 5-hydroxymethylfurfural (HMF) by using methane sulfonic acid as homogeneous catalyst and sodium chloride as promoter, a series of experiments are carried out.

### Example 1: 5-hydroxymethyl furfural (HMF) degradation study

A study is conducted to evaluate HMF degradation. All experiments are carried out in batch operation by using 100 ml Parr autoclave reactor equipped with pitch blade turbine impeller for agitation. The temperature is maintained at about + 1 °C of the desired value by PID controller. The reactor is charged with specific amount of sodium chloride (about 0 g, 0.93 g & 1.87 g i.e. about 0 wt %, 1.25 wt % & 2.5 wt % with respect to the total reaction volume), about 0.45 g of methane sulfonic acid (MSA), and about 75 ml of acetone and distilled water mixture (at about 7:3 ratio). The reaction mixture is agitated at about 300 rpm and the reactor is heated to a temperature of about 125 °C for about 30 minutes. Thereafter, the reactor is cooled to room temperature. Percentage decomposition of 5-hydroxymethyl furfural (HMF) is calculated by HPLC analysis. The samples are diluted with deionised water and analysed by using 5µ Phenomenex Nucleosil C18 column. The mobile phase is 10% acetonitrile in water with the flow rate of 1 ml/min and ultra violet detector. The effect of increase in sodium chloride concentration on HMF decomposition is studied and the results are presented in 'Table 1'. HMF undergoes polymerization and decomposition to form sticky solid humin material which are composed of polyethers, levulinic acid, formic acid, decomposed and carbonised sugars. However, rehydration of HMF leads to the formation of levulinic acid and formic acid.

| Table 1 | | |
|---|---|---|
| Experiment No. | NaCl (wt % w.r.t. to total reaction volume) | % HMF decomposition (wt) |
| 1 | Without NaCl | 18 |
| 2 | 1.25 | 12 |
| 3 | 2.5 | 2 |

As observed from Table 1, an increase in promoter (NaCl) concentration decreases the % HMF decomposition thus establishing the positive effect of promoter. This increase in HMF yield is due to solvation effect.

### Example 2: Effect of sugar concentration on HMF yield

All experiments are carried out in batch operation by using 100 ml Parr autoclave reactor equipped with pitch blade turbine impeller for agitation. The temperature is maintained at + 1 °C of the desired value by PID controller. The reactor is charged with specific amount of fructose (about 7.5 g, 11.25 g and 15.0 g, i.e. about 10 wt%, 15 wt%, 20 wt% w.r.t. to total reaction volume), about 0.45 g of methane sulfonic acid (MSA), about 75 ml of acetone and distilled water mixture (at about 7:3 ratio), and about 1.87 g of sodium chloride. The reaction mixture is agitated at about 300 rpm and the reactor is heated to a temperature of about 125 °C for about 20 minutes. Then, the reactor is cooled to room temperature. Insoluble humins are not observed in the reaction mixture. Based on fructose reacted, the yield of 5-hydroxymethyl furfural is calculated by HPLC analysis. The samples are diluted with deionised water and analysed by using 5µ Phenomenex Nucleosil C18 column. The mobile phase is 10% acetonitrile in water with the flow rate of 1 ml/min and ultra violet detector. The effect of increase in fructose concentration on HMF yield is studied and results are presented in 'Table 2'.

| Table 2 | | | |
|---|---|---|---|
| Experiment No. | Fructose (wt% w.r.t. to total reaction volume) | % Conversion (fructose) | %Yield (HMF) |
| 4 | 10 | 98 | 84 |
| 5 | 15 | 97 | 85 |
| 6 | 20 | 98 | 74 |

As seen from Table 2, high yield of HMF is obtained (about 85 %) even with an increased fructose concentration of 15 %. Thus, the process of present disclosure is successful in achieving improved HMF yield even at high sugar (monosaccharide) concentration.

### Example 3: Effect of promoter concentration on sugar conversion and HMF yield

The effect of increase in sodium chloride concentrations on fructose conversion and HMF yield is studied and results are presented in 'Table 3'. All experiments are carried out in batch operation by using 100 ml Parr autoclave reactor. The temperature is maintained at + 1°C of the desired value by PID controller. The reactor is charged with specific amount of sodium chloride (about 0 g, 0.46 g, 0.93 g & 1.87 g i.e. about 0 wt%, 0.62 wt%, 1.25 wt% & 2.5 wt% w.r.t. to total reaction volume), about 11.25 g of fructose, about 0.45 g of methane sulfonic acid (MSA), and about 75 ml of acetone and distilled water mixture (7:3 ratio). The reaction mixture is agitated at about 300 rpm and the reactor is heated to a temperature of about 125 °C for about 20 minutes. Then, the reactor is cooled to room temperature. Insoluble humins are not observed in the reaction mixture. Based on fructose reacted, the yield of 5-hydroxymethyl furfural is calculated by HPLC analysis as described in Example 1.

| Table 3 | | | |
|---|---|---|---|
| Experiment No. | NaCl (wt% w.r.t. to total reaction volume) | % Conversion (fructose) | %Yield (HMF) |
| 7 | Without NaCl | 80 | 72 |
| 8 | 0.62 | 88 | 80 |
| 9 | 1.25 | 96 | 82 |
| 10 | 2.5 | 97 | 85 |

As observed from Table 3, high % conversion of fructose and high yield of HMF is obtained with increasing concentrations of promoter. In particular, addition of promoter (NaCl) improves HMF yield from 72% to 85%, and fructose conversion from 80% to 97%.

### Example 4: Effect of temperature on sugar conversion and HMF yield

The effect of temperature on fructose conversion and HMF yield is studied and results are presented in 'Table 4'. All experiments are carried out in batch operation by using 100 ml Parr autoclave reactor. The temperature is maintained at + 1 °C of the desired value by PID controller. The reactor is charged with about 11.25 g of fructose, about 1.87 g of sodium chloride, about 0.45 g of methane sulfonic acid, and about 75 ml of acetone and distilled water mixture (at a ratio of about 7:3). The reaction mixture is agitated at about 300 rpm and the reactor is heated to specific temperatures for about 20 minutes. Thereafter, the reactor is cooled to room temperature. Insoluble humins are not observed in the reaction mixture. Based on the fructose reacted, the yield of 5-hydroxymethyl furfural is calculated by HPLC analysis as described in Example 1.

| Table 4 | | | |
|---|---|---|---|
| Experiment No. | Temperature (°C) | % Conversion (fructose) | %Yield (HMF) |
| 11 | 115 | 85 | 61 |
| 12 | 120 | 91 | 84 |
| 13 | 125 | 97 | 86 |
| 14 | 130 | 100 | 67 |

As observed from Table 4, HMF yield is increased with increase in temperature from 115 °C to 125 °C. However, beyond 125 °C, HMF yield is decreased due to thermal degradation of HMF.

### Example 5: Effect of solvent system on sugar conversion and HMF yield

The effects of solvent mixtures (mixture of acetone and water) at varying ratios on fructose conversion and HMF yield are studied and the results are presented in 'Table 5'. All experiments are carried out in batch operation by using 100 ml Parr autoclave reactor. The temperature is maintained at about + 1 °C of the desired value by PID controller. The reactor is charged with about 11.25 g of fructose, about 1.87 g of sodium chloride, about 0.45 g of methane sulfonic acid, and about 75 ml of acetone and distilled water mixture (at ratios of about 7:3, 5:5, 3:7 & 0:10 respectively). The reaction mixture is agitated at about 300 rpm and reactor is heated to a temperature of about 125 °C for about 20 minutes. Then, the reactor is cooled to room temperature. Insoluble humins are not observed in the reaction mixture. Based on fructose reacted, the yield of 5-hydroxymethyl furfural is calculated by HPLC analysis as described in Example 1.

| Table 5 | | | |
|---|---|---|---|
| Experiment No. | Acetone: water composition (vol/vol) | % Conversion (fructose) | %Yield (HMF) |
| 15 | 7:3 | 97 | 85 |
| 16 | 5:5 | 87 | 76 |
| 17 | 3:7 | 80 | 65 |
| 18 | 100% water | 76 | 40 |

As seen from Table 5, when the amount of acetone is increased in solvent system for HMF synthesis, the percentage fructose conversion and HMF yield are also increased from 40 % to about 85%. Further, a mixture of acetone and water at a ratio of about 7:3 results in 97 % fructose conversion and as high as 85 % HMF yield.

### Example 6: Effect of homogeneous catalyst concentration on sugar conversion and HMF yield

The effect of increase in methane sulfonic acid (MSA) concentration on fructose conversion and HMF yield is studied and the results are presented in 'Table 6'. All experiments are carried out in batch operation by using 100 ml Parr autoclave reactor. The temperature is maintained at about + 1 °C of the desired value by PID controller. The reactor is charged with specific amounts of MSA (about 0.30 g, 0.45 g & 0.60 g, i.e. about 0.4 wt%, 0.6 wt% & 0.8 wt% w.r.t. to total reaction volume), about 11.25 g of fructose, about 1.87 g of sodium chloride, and about 75 ml of acetone and distilled water mixture (at a ratio of about 7:3). The reaction mixture is agitated at about 300 rpm and the reactor is heated to about 125 °C for about 20 minutes. Thereafter, the reactor is cooled to room temperature. Insoluble humins are not observed in the reaction mixture. Based on fructose reacted, the yield of 5-hydroxymethyl furfural is calculated by HPLC analysis as described in Example 1.

| Table 6 | | | |
|---|---|---|---|
| Experiment No. | MSA (wt% w.r.t. to total reaction volume) | % Conversion (fructose) | %Yield (HMF) |
| 19 | 0.4 | 95 | 86 |
| 20 | 0.6 | 97 | 85 |
| 21 | 0.8 | 98 | 84 |

As observed from Table 6, the use of homogeneous catalyst (MSA) result in high % fructose conversion and HMF yield. It is also observed that with increase in MSA concentration, HMF yield is not significantly affected.

### Example 7: Effect of various salts on sugar conversion and HMF yield

The effect of various salts (sodium chloride and potassium chloride) on fructose conversion and HMF yield is studied and the results are presented in 'Table 7'. All experiments are carried out in batch operation by using 100 ml Parr autoclave reactor. The temperature is maintained at about + 1 °C of the desired value by PID controller. The reactor is charged with about 1.87 g of salts (sodium chloride and potassium chloride), about 11.25 g of fructose, about 0.45 g of MSA, and about 75 ml of acetone and distilled water mixture (at a ratio of about 7:3). The reaction mixture is agitated at about 300 rpm and the reactor is heated to about 125 °C for about 20 minutes. Thereafter, the reactor is cooled to room temperature. Based on fructose reacted, the yield of 5-hydroxymethyl furfural is calculated by HPLC analysis as described in Example 1.

| Table 7 | | | |
|---|---|---|---|
| Experiment No. | Salts | % Conversion (fructose) | %Yield (HMF) |
| 22 | Potassium chloride | 98 | 80 |
| 23 | Sodium chloride | 97 | 84 |

The effect of inorganic salts such as potassium chloride and sodium chloride are studied. The presence of sodium chloride or potassium chloride results in higher HMF yield.

### Example 8: Effect of various solvent systems on sugar conversion and HMF yield

The effect of solvents (acetone: water, methanol: water and isopropyl alcohol: water) on fructose conversion and HMF yield is studied and the results are presented in 'Table 8'. All experiments are carried out in batch operation by using 100 ml Parr autoclave reactor. The temperature is maintained at about + 1 °C of the desired value by PID controller. The reactor is charged with about 1.87 g of sodium chloride, about 11.25 g of fructose, about 0.45 g of MSA, and about 75 ml of organic solvent (acetone, methanol and isopropyl alcohol) and distilled water mixture (at a ratio of about 7:3). The reaction mixture is agitated at about 300 rpm and the reactor is heated to about 125 °C for about 20 minutes. Thereafter, the reactor is cooled to room temperature. Based on fructose reacted, the yield of 5-hydroxymethyl furfural is calculated by HPLC analysis as described in Example 1.

| Table 8 | | | |
|---|---|---|---|
| Experiment No. | Solvents | % Conversion (fructose) | %Yield (HMF) |
| 26 | Water | 76 | 40 |
| 27 | Methanol:water (7:3) | 84 | 59 |
| 28 | IPA:water (7:3) | 89 | 70 |
| 29 | Acetone:water (7:3) | 98 | 84 |

It is observed that alcohol containing solvent system gives low HMF yield. However, ketone containing solvent system such as Acetone: water results in high fructose conversion and HMF yield respectively.

### Example 9: Synthesis of HMF from fructose

All experiments are carried out in batch operation by using 100 ml Parr autoclave reactor. The temperature is maintained at about + 1 °C of the desired value by PID controller. The reactor is charged with about 1.87 g of sodium chloride, about 11.25 g of fructose), about 0.45 g of MSA, and about 75 ml of acetone, and distilled water mixture (at a ratio of about 7:3). The reaction mixture is agitated at about 300 rpm and the reactor is heated to about 125 °C for about 20 minutes. Thereafter, the reactor is cooled to room temperature. Based on fructose reacted, the yield of 5-hydroxymethyl furfural is calculated by HPLC analysis. Results are presented in 'Table 9'.

| Table 9 | | | |
|---|---|---|---|
| Experiment No. | Sugars | % Conversion (fructose) | %Yield (HMF) |
| 30 | Fructose | 98 | 88 |

### Example 10: 5-hydroxymethyl furfural (HMF) isolation and Solvent extraction procedure

HMF produced in the above example is isolated by adding 0.5 gm of sodium carbonate/sodium bicarbonate is added to the reaction mass to neutralise the MSA. After that HMF is extracted from reaction mass by using 100 ml of ethyl acetate. Then, ethyl acetate is evaporated under reduced pressure in rota vac to obtain isolated 5-hydroxymethyl furfural.

It is observed that dehydration of fructose results in very good HMF yield. Thus, the present disclosure provides a method of synthesis of furfural derivatives, particularly HMF wherein said method showcases numerous advantages including but limited to selective dehydration of monosaccharide to furfural derivative, high monosaccharide conversion, simple & easy procedure of extraction/purification of product, significantly low by-product formation, mild reaction/operating conditions and devoid of high boiling solvent(s), reduced process time, and high product yield.

The present disclosure in view of the above described illustrations and various embodiments, is thus able to overcome the various deficiencies of prior art and provide for an improved process for synthesizing furfural derivatives including but not limited to HMF.

Additional embodiments and features of the present disclosure will be apparent to one of ordinary skill in art based on the description provided herein. The embodiments herein provide various features and advantageous details thereof in the description. Descriptions of well-known/conventional methods and techniques are omitted so as to not unnecessarily obscure the embodiments herein.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments in this disclosure have been described in terms of preferred embodiments, those skilled in the art will recognize that the embodiments herein can be practiced with modification within the spirit and scope of the embodiments as described herein.

Throughout this specification, the word "comprise", or variations such as "comprises" or "comprising" wherever used, will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the disclosure to achieve one or more of the desired objects or results.

As used herein, the terms "promoter" and "co-catalyst" are employed interchangeably within the instant disclosure and refer to the composition of the instant disclosure.

Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

While considerable emphasis has been placed herein on the particular features of this disclosure, it will be appreciated that various modifications can be made, and that many changes can be made in the preferred embodiments without departing from the principles of the disclosure. These and other modifications in the nature of the disclosure or the preferred embodiments will be apparent to those skilled in the art from the disclosure herein, whereby it is to be distinctly understood that the foregoing descriptive matter is to be interpreted merely as illustrative of the disclosure and not as a limitation.

## Claims

1. A process for preparing furfural or derivatives thereof, said process comprising act of dehydrating monosaccharide in presence of organic acid catalyst and promoter.

2. The process as claimed in claim 1, wherein the furfural is 5-hydroxymethylfurfural.

3. The process as claimed in claim 1, wherein the monosaccharide is fructose.

4. The process as claimed in claim 1, wherein the organic acid catalyst is organosulfur compound, preferably alkylsulfonic acid.

5. The process as claimed in claim 1, wherein the organic acid catalyst is methane sulfonic acid.

6. The process as claimed in claim 1, wherein the promoter is salt, and wherein said salt is metal halide.

7. The process as claimed in claim 1, wherein the promoter is alkali metal halide of formula I
M⁺X⁻ Formula I
wherein,
'M' is an alkali metal selected from a group comprising sodium, potassium, lithium, rubidium, and caesium; and
'X' is a halide selected from a group comprising fluoride, chloride, bromide and iodide.

8. The process as claimed in claim 1, wherein the process is carried out in presence of solvent, and wherein said solvent is selected from a group comprising polar aprotic solvent and polar protic solvent, or a combination thereof.

9. The process as claimed in claim 8, wherein the polar aprotic solvent is selected from a group comprising acetone, tetrahydrofuran, ethyl acetate, dimethylformamide, acetonitrile, dimethyl sulfoxide, preferably acetone; and wherein the polar protic solvent is selected from a group comprising water, methanol, ethanol, preferably water.

10. The process as claimed in claim 8, wherein the solvent is a combination of polar aprotic solvent and polar protic solvent, preferably acetone:water.

11. The process as claimed in claim 1, wherein the monosaccharide is a concentration ranging from about 1 to 30 wt% , preferably about 10 to 15 wt%; organic acid catalyst is at a concentration ranging from about 0.1 to 1.5% wt%, preferably about 0.4 to 0.8 wt%; the promoter is at a concentration ranging from about 0.1 to 5% wt%, preferably about 1.25 to 2.5 wt% ; and the polar aprotic solvent and the polar protic solvent are at a ratio ranging from about 9:1 to 1:9, preferably about 7:3.

12. The process as claimed in claim 1, wherein said process is carried out in a batch operation at a temperature ranging from about 115°C to 130°C, preferably about 125°C, and for a time period ranging from about 15 minutes to 30 minutes, preferably about 15 minutes to 20 minutes.

13. The process as claimed in claim 1, wherein the process further comprises act of isolation and optional purification of the synthesized furfural or derivatives thereof; and wherein said isolation and purification is carried out by acts selected from a group comprising addition of base, quenching, solvent extraction, filtration, evaporation and crystallization, or any combination of acts thereof, preferably solvent extraction using ethyl acetate.

14. The process as claimed in claim 1, wherein conversion of the monosaccharide ranges from about 70 % to 100 %, preferably about 80 % to 98 %, and the yield of furfural or its derivative ranges from about 65 % to 88 %, preferably about 88%.
